# EUROPEAN PATENT SPECIFICATION

(11) **EP 4 134 088 B1**
(45) Date of publication and mention of the grant of the patent: **01.07.2026**
(21) Application number: 22756056.2
(22) Date of filing: 09.02.2022
(51) Int. Cl.: A61K 35/28, A61K 35/51, A61P 3/10, C12N 5/0775

(54) **MESENCHYMAL STEM CELLS FOR USE IN TREATING DIABETES CAUSED BY IMMUNE-CHECKPOINT INHIBITORS**
MESENCHYMALE STAMMZELLEN ZUR VERWENDUNG BEI DER BEHANDLUNG EINES DURCH IMMUN-CHECKPOINT-INHIBITOREN INDUZIERTEN DIABETES
CELLULES SOUCHES MÉSENCHYMATEUSES POUR UTILISATION DANS LE TRAITEMENT D'UN DIABÈTE INDUIT PAR DES INHIBITEURS DES POINTS DE CONTRÔLE IMMUNITAIRES

(30) Priority: 22.02.2021 JP 2021026666
(43) Date of publication of application: 15.02.2023
(73) Proprietor: Rohto Pharmaceutical Co., Ltd., Osaka-shi, Osaka 544-8666 (JP); OSAKA UNIVERSITY, Suita-shi Osaka 565-0871 (JP)
(72) Inventor: KITA Shunbun, Suita-shi, Osaka 565-0871 (JP); HORITANI Emi, Suita-shi, Osaka 565-0871 (JP); MAEDA Norikazu, Suita-shi, Osaka 565-0871 (JP); SHIMOMURA Iichiro, Suita-shi, Osaka 565-0871 (JP); NISHIDA Hiroyuki, Osaka-shi, Osaka 544-8666 (JP)
(74) Representative: Mewburn Ellis LLP
(86) International application number: PCT/JP2022/005125
(87) International publication number: WO 2022/176735

(56) References cited:
- WO-A1-2006/090918
- WO-A1-2020/257710
- WO-A2-2009/023566
- JP-A- 2009 535 347
- JP-A- 2020 523 400
- VENETSANAKI V. ET AL: "Diabetes Mellitus Secondary to Treatment with Immune Checkpoint Inhibitors", vol. 26, no. 1, 1 February 2019 (2019-02-01), pages 111 - 114, XP055838120, Retrieved from the Internet <URL:http://current-oncology.com/index.php/oncology/article/viewFile/4151/3323> DOI: 10.3747/co.26.4151
- CLOTMAN KATRIEN, JANSSENS KATLEEN, SPECENIER POL, WEETS ILSE, DE BLOCK CHRISTOPHE E M: "Programmed Cell Death-1 Inhibitor–Induced Type 1 Diabetes Mellitus", JOURNAL OF CLINICAL ENDOCRINOLOGY AND METABOLISM, THE ENDOCRINE SOCIETY, US, vol. 103, no. 9, 1 September 2018 (2018-09-01), US , pages 3144 - 3154, XP055960640, ISSN: 0021-972X, DOI: 10.1210/jc.2018-00728
- IMAGAWA AKIHISA; TACHIBANA MEGUMI: "Fulminant type 1 diabetes: recent research progress and future prospects", DIABETOLOGY INTERNATIONAL, SPRINGER JAPAN, TOKYO, vol. 11, no. 4, 16 September 2020 (2020-09-16), Tokyo , pages 336 - 341, XP037262369, ISSN: 2190-1678, DOI: 10.1007/s13340-020-00466-2
- EMI HORIYA, TOSHIFUMI KITA, YUTO NAKAMURA, SEIRIN HATA, TOMONORI OKITA, SHIRO FUKUDA, YUYA FUJISHIMA, HITOSHI NISHIZAWA, HOICHI MA: "P-3-6 Investigation of novel diabetes treatment by mesenchymal stem cells", TONYOBYO - JOURNAL OF THE JAPAN DIABETES SOCIETY, JAPAN DIABETES SOCIETY, TOKYO, JP, vol. 64, no. Suppl. 1, 30 April 2021 (2021-04-30) - 22 May 2021 (2021-05-22), JP , pages S - 331, XP009539141, ISSN: 0021-437X, DOI: 10.11213/tonyobyo.64.S-330

## Description

### TECHNICAL FIELD

The present invention relates to Mesenchymal stem cells for use in the prevention and/or the treatment of diabetes which is caused by an immune checkpoint inhibitor.

### BACKGROUND ART

Immune checkpoint inhibitors have been applied to various malignancies and have been increasingly used. The Japanese health insurance covers treatment of malignant melanoma with an anti-CTLA-4 antibody ipilimumab (YERVOY^{®}), treatment of malignant melanoma, non-small cell lung cancer, renal cell carcinoma, Hodgkin's lymphoma, head and neck cancer, and gastric cancer with an anti-PD-1 antibody nivolumab (OPDIVO^{®}), treatment of malignant melanoma, non-small cell lung cancer, and urothelial carcinoma with an anti-PD-1 antibody pembrolizumab (KEYTRUDA^{®}), treatment of Merkel cell carcinoma with an anti-PD-L1 antibody avelumab (BAVENCIO^{®}), and treatment of non-small cell lung cancer with an anti-PD-L1 antibody atezolizumab (TECENTRIQ^{®}).

Although these drugs have anti-tumor effect by activating immunoreactions, their mechanisms of action may cause autoimmune diseases, and in fact the onset of various immune-related adverse events has been reported. Endocrinopathy, which is one of the adverse events, has been so far reported including hypopituitarism, adrenocortical insufficiency, thyroid dysfunction, hypoparathyroidism, and type 1 diabetes. According to "Clinical guidelines for endocrine disorders associated with immune checkpoint inhibitors ", withdrawal of the immune checkpoint inhibitor may be required until glucose control with insulin treatment is improved for type 1 diabetes. This is because patients with type 1 diabetes experience irreversible loss of function of pancreatic β cells, a rapid increase in blood glucose, and finally poor prognosis in the absence of any adequate treatment and must be early diagnosed and treated (with insulin) for type 1 diabetes (Non Patent Document 1).

Pathogenic mechanism of diabetes associated with immune checkpoint inhibitors has been elucidated in NOD mice which represent a spontaneous model for type 1 diabetes (Non Patent Document 2). In other words, inhibiting PD-1 with an immune checkpoint inhibitor will activate T cells and macrophages and allow them to attack autologous β cells, leading to the development of type 1 diabetes. Thus, it has been believed that it is difficult to suppress development of type 1 diabetes while maintaining the effect of immune checkpoint inhibitors on malignancies. Under such circumstances, there is a strong desire for a method of treatment that can suppress side effects while maintaining the effect of checkpoint inhibitors.

In recent years, cultured cells derived from tissues and cells in various parts of a human body, and cultured fertilized eggs have been put into practical use for regenerative medicine and other applications. One of the cultured cells is a mesenchymal stem cell. Mesenchymal stem cells, which were isolated from bone marrow by Friedenstein for the first time, are multipotent progenitor cells (see Non Patent Document 3). Mesenchymal stem cells have been revealed to be located in various tissues including bone marrow, umbilical cord, and adipose, and transplantation of mesenchymal stem cells holds promise for a novel treatment for various intractable diseases (see Patent Document 1 to 2). It has recently been found that mesenchymal stromal cells such as in adipose tissue, placenta, umbilical cord, and egg membrane include cells having functions equivalent to mesenchymal stem cells. Accordingly, mesenchymal stem cells may be referred to as mesenchymal stromal cells.

### PRIOR ART DOCUMENTS

### Patent Document

[Patent Document 1] JP 2012-157263 A
[Patent Document 2] JP 2012-508733 A

### Non Patent Document

[Non Patent Document 1] Folia Endocrinologica Japonica, 2018, Vol. 94, Suppl. November, pp. 1-11
[Non Patent Document 2] Proc. Natl. Acad. Sci. USA, 2020, Vol. 49, No. 117, pp. 31319-31330
[Non Patent Document 3] Pittenger F. M. et al., Science, 1999, 284, pp. 143-147

WO 2018 227244 describes a method for treating a side effect of immunotherapy, comprising administering a mesenchymal stem cell.

WO 2007 127408 describes a method of treating diabetes comprising administering isolated multipotent stromal cells. Immune checkpoint inhibitor induced diabetes is not mentioned; that disease is distinct from conventional diabetes.

WO 2009 023566 describes a method of treating new onset type 1 diabetes comprising administering autologous or allogeneic mesenchymal stem cells prior to autoimmune-induced complete depletion of insulin-producing pancreatic beta cells. Immune checkpoint inhibitor induced diabetes is not mentioned; that disease is distinct from conventional diabetes.

### SUMMARY OF INVENTION

### Technical Problem

The present invention has been made in the circumstances as described above and aims to provide a therapeutic agent for diabetes that is highly effective on diabetes developed as a side effect of an administration of immune checkpoint inhibitors and that has a certain effect on many patients.

### Solution to Problem

As a result of the intensive research to solve the problems, the present inventors have found that administering mesenchymal stem cells to patients of diabetes caused by immune checkpoint inhibitors can suppress development of the diabetes and can improve markedly symptoms of the diabetes developed. The present invention has been accomplished on the basis of these findings.
The invention is defined in the appended claims..

### Advantageous Effects of Invention

Administering mesenchymal stem cells of the present invention can suppress development of diabetes associated with immune checkpoint inhibitors and can improve markedly symptoms of diabetes caused by immune checkpoint inhibitors. Because the mesenchymal stem cells will cause less rejection in allogeneic subjects, previously prepared donor cells that have been expanded and cryopreserved can be used as mesenchymal stem cells which are included in the prophylactic and/or therapeutic agents for diabetes of the present invention. Accordingly, such mesenchymal stem cells have an advantage that they are more easily commercialized than using autologous mesenchymal stem cells and that it is easy to achieve a certain stable effect.

### BRIEF DESCRIPTION OF DRAWINGS

[Fig.1] Fig. 1 shows the progression of blood glucose levels in mice administered "anti-PD-L1 antibody (CTRL) "and mice administered"anti-PD-L1 antibody and ADMSC (MSC)".
[Fig. 2] Fig. 2 shows the progression of development of diabetes in mice administered "anti-PD-L1 antibody (CTRL)" and mice administered "anti-PD-L1 antibody and ADMSC (MSC)".
[Fig. 3] Fig. 3 shows the progression of body weights of mice administered "anti-PD-L1 antibody (CTRL)" and mice administered "anti-PD-L1 antibody and ADMSC (MSC)".
[Fig. 4] Fig. 4 shows hematoxylin-eosin staining (left) and insulin immunostaining (right) of the pancreas from a mouse administered "anti-PD-L1 antibody (CTRL)".
[Fig. 5] Fig. 5 shows hematoxylin-eosin staining (left) and insulin immunostaining (right) of the pancreas from a mouse administered "anti-PD-L1 antibody and ADMSC (MSC)".
[Fig. 6] Fig. 6 shows the comparison of islet areas in pancreas sections from mice administered "anti-PD-L1 antibody (CTRL)" and mice administered "anti-PD-L1 antibody and ADMSC (MSC)".
[Fig. 7] Fig. 7 shows the comparison of islet areas in pancreas sections from CNTL group and MSC group that both had no development of diabetes in Example 1.

### DESCRIPTION OF EMBODIMENTS

A prophylactic and/or therapeutic agent for diabetes will be described in detail below.

### Prophylactic and/or therapeutic agents for diabetes

A prophylactic and/or therapeutic agent for diabetes described herein comprises mesenchymal stem cells. Diabetes that can be treated with the prophylactic and/or therapeutic agents for diabetes described herein is diabetes caused by an administration of immune checkpoint inhibitors. The prophylactic and/or therapeutic agents for diabetes can suppress development of diabetes associated with immune checkpoint inhibitors and can improve markedly symptoms of diabetes caused by immune checkpoint inhibitors.

The immune checkpoint inhibitor refers to a therapeutic agent for cancers that targets an "immune checkpoint" suppressing the action of immune cells. The immune checkpoint inhibitor may be any agent that inhibits an immune checkpoint. The immune checkpoint inhibitor includes an agent that inhibits the binding of PD-1 to its ligand (PD-L1, PD-L2), an agent that inhibits the binding of PD-L1 to its receptor PD-1, and an agent that inhibits the binding of CTLA-4 to its ligands B7.1 (CD80) and B7.2 (CD86) molecules on antigen presenting cells. Examples of the immune checkpoint inhibitor include PD-1 antibody, PD-L1 antibody, and cytotoxic T-lymphocyte antigen-4 (CTLA-4) antibody, and specifically Nivolumab (OPDIVO^{®}, ONO PHARMACEUTICAL CO., LTD.), Pembrolizumab (KEYTRUDA^{®}, MSD), Spartalizumab (Novartis Pharma K.K.), Cemiplimab (Sanofi K.K.), Avelumab (BAVENCIO^{®}, Merck Biopharma Co., Ltd), Atezolizumab (TECENTRIQ^{®}, CHUGAI PHARMACEUTICAL CO., LTD.), Durvalumab (IMFINZI^{®}, AstraZeneca K.K.), Ipilimumab (YERVOY^{®}, Bristol Myers Squibb), and Tremelimumab (AstraZeneca K.K.).

### Mesenchymal stem cells

Mesenchymal stem cells in the present invention mean cells that have an ability to differentiate into cells that belong to mesenchymal cells (such as osteocytes, cardiac myocytes, chondrocytes, tenocytes, and adipocytes) and can proliferate while maintaining the ability. The term mesenchymal stem cells as used in the present invention mean the same as mesenchymal stromal cells and are indistinguishable from mesenchymal stromal cells. Examples of tissues comprising mesenchymal stem cells include adipose tissue, umbilical cord, bone marrow, umbilical cord blood, placenta, and dental pulp. Thus, for example, adipose tissue-derived mesenchymal stem cells mean mesenchymal stem cells which are included in adipose tissue and may be referred to as adipose tissue-derived mesenchymal stromal cells. Among these, in terms of efficacy and availability of the prophylactic and/or therapeutic agents for diabetes described herein, mesenchymal stem cells are preferably adipose tissue-derived mesenchymal stem cells, umbilical cord-derived mesenchymal stem cells, bone marrow-derived mesenchymal stem cells, placenta-derived mesenchymal stem cells, and dental pulp-derived mesenchymal stem cells, more preferably adipose tissue-derived mesenchymal stem cells, umbilical cord-derived mesenchymal stem cells, and bone marrow-derived mesenchymal stem cells, even more preferably adipose tissue-derived mesenchymal stem cells and umbilical cord-derived mesenchymal stem cells, and particularly preferably umbilical cord-derived mesenchymal stem cells.

The mesenchymal stem cells in the present invention are preferably autologous or allogeneic to a subject. Because the mesenchymal stem cells will cause less rejection in allogeneic subjects, previously prepared donor cells that have been expanded and cryopreserved can be used as mesenchymal stem cells which are included in the prophylactic and/or therapeutic agents for diabetes described herein. Accordingly, such mesenchymal stem cells become commercial more easily than prepared autologous mesenchymal stem cells and easily achieve a certain stable effect. Thus, the mesenchymal stem cells in the present invention are preferably allogeneic.

The mesenchymal stem cells in the present invention mean any cell population comprising mesenchymal stem cells. At least 20% or more, preferably 30%, 40%, 50%, 60%, 70%, 75%, 80%, 85%, 90%, 93%, 96%, 97%, 98%, or 99% or more of the cell population is mesenchymal stem cells.

The adipose tissue in the present invention means a tissue comprising adipocytes and mesenchymal stromal cells including microvascular cells and is, for example, a mammalian subcutaneous adipose obtained by surgical excision or suction. The adipose tissue can be obtained from subcutaneous adipose. The adipose tissue is preferably obtained from the same animal species as an individual to which adipose tissue-derived mesenchymal stem cells as described below are administered. Considering that the adipose tissue-derived mesenchymal stem cells are administered to human, the adipose tissue is more preferably human subcutaneous adipose. The subcutaneous adipose may be derived from a living or dead individual. The adipose tissue to be used in the present invention is preferably taken from a living individual. When taken from an individual, the subcutaneous adipose is sucked by, for example, power-assisted liposuction (PAL), elcornia laser liposuction, or body jet liposuction. To maintain cell status, no ultrasound is preferably used.

The umbilical cord in the present invention is a white tubular tissue connecting the fetus to the placenta, is composed of umbilical vein, umbilical artery, gelatinous tissue (Wharton's Jelly), umbilical cord matrix, and the like, and includes many mesenchymal stem cells. The umbilical cord is preferably obtained from the same animal species as a subject for which the prophylactic and/or therapeutic agents for diabetes described herein are used. Considering that the prophylactic and/or therapeutic agents for diabetes are administered to human, the umbilical cord is more preferably human umbilical cord.

Bone marrow in the present invention refers to a soft tissue filling the inside of bone cavities and is a hematopoietic organ. The bone marrow fluid is found in bone marrow, and cells that are present in the bone marrow fluid are called bone marrow cells. Bone marrow cells include erythrocytes, granulocytes, megakaryocytes, lymphocytes, and adipocytes as well as mesenchymal stem cells, hematopoietic stem cells, and vascular endothelial progenitor cells. Bone marrow cells can be taken from, for example, human ilium, long bone, or other bones.

In the present invention, tissue-derived mesenchymal stem cells such as adipose tissue-derived mesenchymal stem cells, umbilical cord-derived mesenchymal stem cells, and bone marrow-derived mesenchymal stem cells mean any cell population comprising tissue-derived mesenchymal stem cells such as adipose tissue-derived mesenchymal stem cells, umbilical cord-derived mesenchymal stem cells, and bone marrow-derived mesenchymal stem cells, respectively. At least 20% or more, preferably 30%, 40%, 50%, 60%, 70%, 75%, 80%, 85%, 90%, 93%, 96%, 97%, 98%, or 99% or more of the cell population is tissue-derived mesenchymal stem cells such as adipose tissue-derived mesenchymal stem cells, umbilical cord-derived mesenchymal stem cells, and bone marrow-derived mesenchymal stem cells.

The mesenchymal stem cells in the present invention may be characterized, for example, for growth characteristics (e.g., population doubling capability and doubling time from passage to senescence), by karyotype analysis (e.g., normal karyotype, maternal karyotype, or neonatal karyotype), for surface marker expression by flow cytometry (e.g., FACS analysis), by immunohistochemistry and/or immunocytochemistry (e.g., epitope detection), for gene expression profiling (e.g., by gene chip array; polymerase chain reactions such as reverse-transcription PCR, real-time PCR, and traditional PCR), for miRNA expression profiling, by protein array, for secretion of proteins such as cytokines (e.g., by plasma coagulation analysis, ELISA, and cytokine array), for metabolic products (by metabolome analysis), and by other methods known in the art.

### Methods of preparing mesenchymal stem cells

The mesenchymal stem cells in the present invention can be prepared by any methods well known to those skilled in the art. As an example, a method of preparing adipose tissue-derived mesenchymal stem cells will be described below. The adipose tissue-derived mesenchymal stem cells may be obtained by a production method, such as described in U.S. Patent No. 6,777,231, and can be produced, for example, by a method comprising the following steps (i) to (iii):
(i) enzymatically digesting adipose tissue to obtain a cell suspension;
(ii) precipitating the cells and resuspending the precipitated cells in a suitable medium; and
(iii) culturing the cells on a solid surface and removing cells that are unbound to the solid surface.

To confirm that the selected cells are adipose tissue-derived mesenchymal stem cells in the present invention, they may be analyzed for their surface antigens by traditional methods such as flow cytometry. Additionally, the cells may be tested for an ability to differentiate into various cell lineages. Such differentiation may be performed by any traditional method.

The mesenchymal stem cells in the present invention can be prepared as described above and may be defined as cells having the following properties:
(1) they are adhesive to a plastic surface under culture conditions in a standard medium;
(2) they are positive for surface antigens CD44, CD73, and CD90 and negative for CD31, CD34, and CD45; and
(3) they can differentiate into osteocytes, adipocytes, and chondrocytes under culture conditions.

The mesenchymal stem cells of the present invention may be in any state and, for example, may be obtained by detaching the cells in culture and collecting the detached cells or may be frozen in a cryopreservation solution. The mesenchymal stem cells to be used are preferably aliquoted and cryopreserved from the cells expanded at the same time, in terms of stable efficacy and better handling. The cryopreserved mesenchymal stem cells may be thawed just before use and then administered directly as a cell suspension in the cryopreservation solution or administered after suspending the thawed cells in an infusion solution or a medium. The thawed cells may be also mixed with an infusion solution or a medium after removing the cryopreservation solution by any method such as centrifugation.

The therapeutic agents for diabetes can be used to treat diabetes. In particular, the therapeutic agents can be used suitably to treat diabetes caused by an administration of immune checkpoint inhibitors, and more suitably for diabetes caused by anti-PD-1 antibody or anti-PD-L1 antibody administered. The preventive agents for diabetes can be also used to prevent diabetes. In particular, the preventive agents can be used suitably as a prophylactic agent for development of diabetes associated with immune checkpoint inhibitors, and more suitably as a prophylactic agent for diabetes caused by anti-PD-1 antibody or anti-PD-L1 antibody administered.

The preferred route of administration of the prophylactic and/or therapeutic agents for diabetes is an intravenous route, an intraarterial route, or an intramuscular route in view of efficacy.

A dose (dosage) of mesenchymal stem cells in a prophylactic and/or therapeutic agent for diabetes may vary depending on conditions of a patient (such as a body weight, age, symptoms, and physical conditions) and the dosage form of the prophylactic and/or therapeutic agent for diabetes. More mesenchymal stem cells tend to be preferably administered in terms of adequate therapeutic efficacy of the prophylactic and/or therapeutic agents for diabetes, whereas less mesenchymal stem cells tend to be preferably administered in terms of the suppression of side effect occurrence. Typically, for the administration to adults, the number of mesenchymal stem cells to be administered is 1 x 10³ to 1 x 10¹² cells/dose, preferably 1 x 10⁴ to 1 x 10¹¹ cells/dose, more preferably 1 x 10⁵ to 1 x 10¹⁰ cells/dose, and even more preferably 5 x 10⁶ to 1 x 10⁹ cells/dose. The amount of mesenchymal stem cells to be administered per patient's body weight is 1 x 10 to 5 x 10¹⁰ cells/kg, preferably 1 x 10² to 5 x 10⁹ cells/kg, more preferably 1 x 10³ to 5 x 10⁸ cells/kg, and even more preferably 1 x 10⁴ to 5 x 10⁷ cells/kg. This amount of mesenchymal stem cells may be administered more than once as a single dose or may be divided into multiple doses.

The prophylactic and/or therapeutic agents for diabetes described herein may be used prophylactically before the development of diabetes or may be used for the purpose of treatment after the development of diabetes. In other words, the prophylactic and/or therapeutic agents for diabetes may be used for patients in combination with an immune checkpoint inhibitor or may be used after the development of diabetes caused by an administration of immune checkpoint inhibitors.

The prophylactic and/or therapeutic agents for diabetes may be administered in combination with one or more than one other agent. Such other agents include any agents that can be used as a prophylactic and/or therapeutic agent for diabetes. The administration of the prophylactic and/or therapeutic agent for diabetes in combination with one or more than one other agent includes various administrations such as simultaneous administration of the prophylactic and/or therapeutic agent for diabetes and other agents, administration of either of them followed by the other after a certain time, and a combination thereof.

### Methods of preparing the prophylactic and/or therapeutic agents for diabetes

The prophylactic and/or therapeutic agents for diabetes described herein are obtained by mixing mesenchymal stem cells prepared by the preparation method as described above and a suitable liquid for suspending the cells (including pharmaceutically acceptable carriers or additives), according to conventional methods corresponding to their dosage forms.

### Kits for preventing and/or treating diabetes

Described herein is a kit for treating diabetes comprising the prophylactic and/or therapeutic agent for diabetes described above, a container, and a label. The suitable container included in the kit is not particularly limited but includes, for example, a cryotube for freezing mesenchymal stem cells, a container for suspending mesenchymal stem cells, a vial, and a test tube. These containers may be made of various materials such as glass, metal, plastic, or a combination thereof. The labels on the containers indicate the description of contents.

The kit can include other materials desirable from a commercial and user standpoint, including other additives, other agents, diluents, filters, needles, syringes, and package inserts with instructions for use.

### Methods of preventing and/or treating diabetes

The methods of preventing and/or treating diabetes described herein are characterized by the administration of mesenchymal stem cells. Diabetes that can be treated by the methods of preventing and/or treating diabetes described herein is diabetes caused by an administration of immune checkpoint inhibitors. The methods of preventing and/or treating diabetes described herein can suppress development of diabetes associated with immune checkpoint inhibitors and can improve markedly symptoms of the diabetes caused by immune checkpoint inhibitors. The methods of preventing and/or treating diabetes described herein comprise administering a prophylactic and/or therapeutic agent for diabetes described herein to an individual. For specific description of the methods, reference should be made to the section describing the prophylactic and/or therapeutic agents for diabetes.

### EXAMPLES

The present invention will be described in detail in the following Examples, but the present invention is not limited to these Examples.

### Effect of MSC on anti-PD-L1 antibody-induced diabetes - 1

### Example 1

An anti-PD-L1 antibody was intraperitoneally administered to mice (NOD/ShiJcl, male, 10 week old, n = 31) on day 0 at a dose of 1,000 µg and on days 2, 5, 7, 9, and 12 at a dose of 500 µg. Adipose-derived stem cells (Lonza, hereinafter referred to as "MSC") suspended in DMEM (Low Glucose, P/S) were administered to mice in MSC group via the tail vein at 1.0 x 10⁶ cells/body on days 0, 2, 5, 7, 9, and 12. Mice in CNTL group administered only DMEM on days 0, 2, 5, 7, 9, and 12. The blood glucose level was measured with a simple blood glucose meter (Dexter). When the blood glucose level was beyond the measurement limit, it was considered 600mg/dl for calculation. Incidence rate of diabetes was calculated by determining that diabetes developed when the blood glucose level was 250 mg/dl or higher.

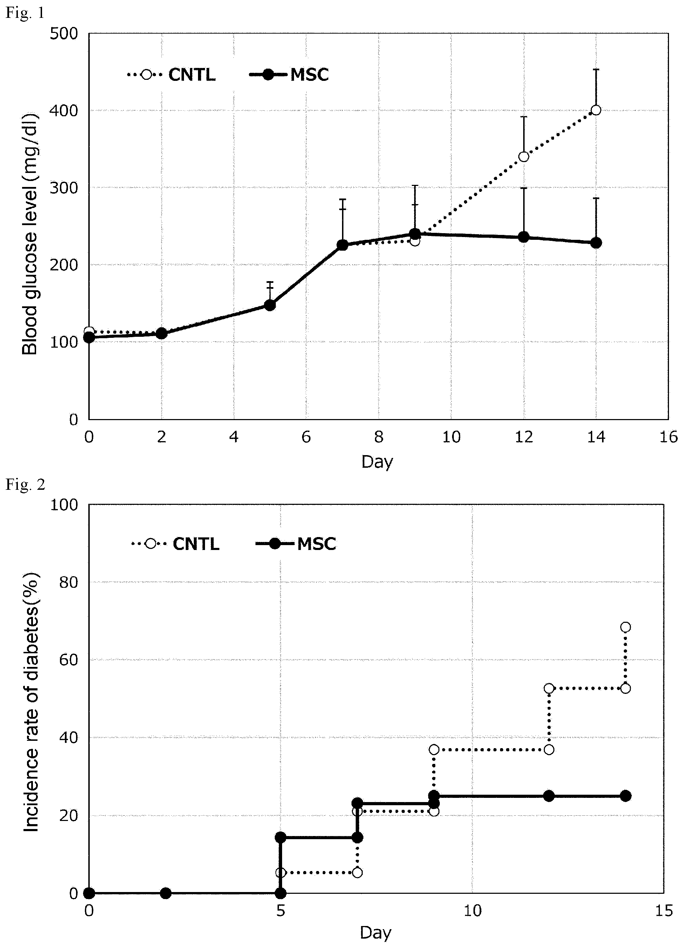

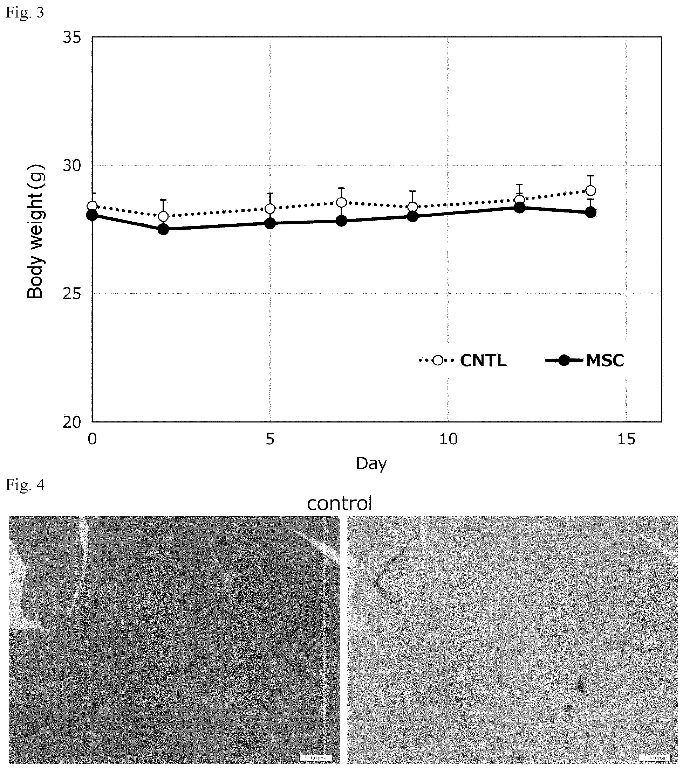

As shown in Fig. 1, it was confirmed that the administration of MSC superiorly suppressed an increase in blood glucose level on day 14. The administration of MSC also successfully suppressed development of diabetes (Fig. 2). It should be noted that mice in CNTL group and MSC group had no decrease in their body weight, and no difference was seen between the progression of body weights of mice in the two groups (Fig. 3).

The results revealed that the administration of MSC can suppress an increase in blood glucose levels caused by administering anti-PD-L1 antibody and the development of diabetes. It is found that mesenchymal stem cells have superior therapeutic effect on diabetes caused by administration of an immune checkpoint inhibitor.

### Effect of MSC on anti-PD-L1 antibody-induced diabetes - 2

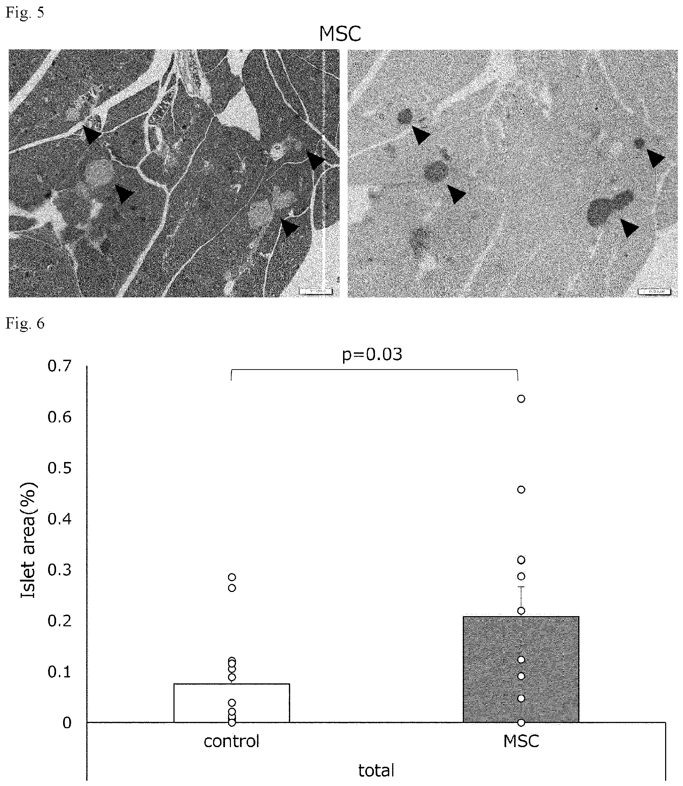

The pancreases from CNTL group and MSC group in Example 1 were subjected to hematoxylin-eosin staining and insulin immunostaining (Figs. 4 and 5). The arrows in Fig. 5 point to pancreatic islets. Pancreatic islets were not seen in Control group, whereas pancreatic islets were clearly seen in MSC group.

### Effect of MSC on anti-PD-L1 antibody-induced diabetes - 3

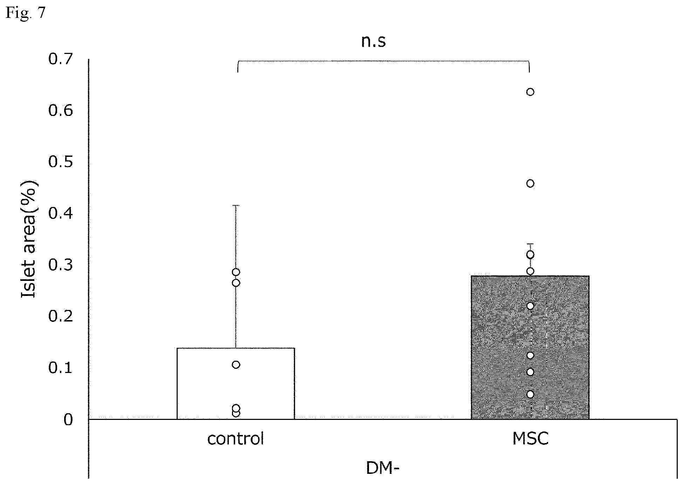

Islet areas in pancreas sections from CNTL group and MSC group in Example 1 were compared (Fig. 6). The islet area in MSC group was significantly larger than that in CNTL group. The islet areas in CNTL group (n = 5) and MSC group (n = 9) that both had no development of diabetes in Example 1 were compared (Fig. 7). The islet area in MSC group that had no development of diabetes also tended to be larger than that in CNTL group that had no development of diabetes.

### INDUSTRIAL APPLICABILITY

The prophylactic and/or therapeutic agents for diabetes described herein can suppress development of diabetes associated with immune checkpoint inhibitors and can improve markedly symptoms of the diabetes caused by immune checkpoint inhibitors. Because the mesenchymal stem cells will cause less rejection in allogeneic subjects, donor cells that have been previously confirmed to have efficacy and have been expanded and cryopreserved can be used as mesenchymal stem cells which are included in the prophylactic and/or therapeutic agents for diabetes of the present invention. Accordingly, such mesenchymal stem cells become commercial more easily than prepared autologous mesenchymal stem cells and have an advantage that it is easy to achieve a certain stable effect.

## Claims

1. Mesenchymal stem cells for use in the prevention and/or the treatment of diabetes which is caused by an immune checkpoint inhibitor.

2. The mesenchymal stem cells for use according to claim 1, wherein the diabetes is diabetes caused by an anti-PD-1 antibody or an anti-PD-L1 antibody.

3. The mesenchymal stem cells for use according to claim 1 or 2, wherein the mesenchymal stem cells are allogeneic to a subject.

4. The mesenchymal stem cells for use according to any of claims 1 to 3, wherein the mesenchymal stem cells are derived from adipose, umbilical cord, or bone marrow.

## Patentansprüche

1. Mesenchymale Stammzellen zur Verwendung bei der Prävention und/oder Behandlung von Diabetes, der durch einen Immuncheckpoint-Inhibitor verursacht wird.

2. Mesenchymale Stammzellen zur Verwendung nach Anspruch 1, wobei der Diabetes Diabetes ist, der durch einen Anti-PD-1-Antikörper oder einen Anti-PD-L1-Antikörper verursacht wird.

3. Mesenchymale Stammzellen zur Verwendung nach Anspruch 1 oder 2, wobei die mesenchymalen Stammzellen allogen für ein Individuum sind.

4. Mesenchymale Stammzellen zur Verwendung nach einem der Ansprüche 1 bis 3, wobei die mesenchymalen Stammzellen von Fettgewebe, der Nabelschnur oder dem Knochenmark abgeleitet sind.

## Revendications

1. Cellules souches mésenchymateuses pour utilisation dans la prévention et/ou le traitement du diabète qui est causé par un inhibiteur de point de contrôle immunitaire.

2. Cellules souches mésenchymateuses pour utilisation selon la revendication 1, dans lesquelles le diabète est un diabète causé par un anticorps anti-PD-1 ou un anticorps anti-PD-L1.

3. Cellules souches mésenchymateuses pour utilisation selon la revendication 1 ou 2, dans lesquelles les cellules souches mésenchymateuses sont allogéniques à un sujet.

4. Cellules souches mésenchymateuses pour utilisation selon l'une quelconque des revendications 1 à 3, dans lesquelles les cellules souches mésenchymateuses sont dérivées de tissu adipeux, de cordon ombilical ou de moelle osseuse.
